(19) Europäisches Patentamt
European Patent Office
Office européen des brevets

(11) Veröffentlichungsnummer : **0 061 709**
**B1**

(12) **EUROPÄISCHE PATENTSCHRIFT**

(45) Veröffentlichungstag der Patentschrift : **05.06.85**

(21) Anmeldenummer : **82102444.5**

(22) Anmeldetag : **24.03.82**

(51) Int. Cl.⁴ : **C 07 D223/20**, C 07 D401/06, C 07 D401/12, C 07 D403/12, C 07 D403/06, C 07 D413/12, A 61 K 31/55// C07F9/65

(54) **5,6-Dihydro-11-alkylen-morphantridin-6-one, ihre Herstellung und diese enthaltende Arzneimittel.**

(30) Priorität : **01.04.81 DE 3113094**

(43) Veröffentlichungstag der Anmeldung : **06.10.82 Patentblatt 82/40**

(45) Bekanntmachung des Hinweises auf die Patenterteilung : **05.06.85 Patentblatt 85/23**

(84) Benannte Vertragsstaaten : **AT BE CH DE FR GB IT LI LU NL SE**

(56) Entgegenhaltungen :
**EP-A- 0 019 157**
**DE-A- 2 724 478**
**FR-M- 3 348**
**GB-A- 1 121 372**
**NL-A- 6 717 154**

(73) Patentinhaber : **BASF Aktiengesellschaft**
**Carl-Bosch-Strasse 38**
**D-6700 Ludwigshafen (DE)**

(72) Erfinder : **Steiner, Gerd, Dr.**
**Oberer Waldweg 1**
**D-6719 Kirchheim (DE)**
Erfinder : **Friedrich, Ludwig, Dr.**
**Nibelungenstrasse 8A**
**D-6831 Bruehl (DE)**
Erfinder : **Lenke, Dieter, Dr.**
**Kekuleplatz 1**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 061 709 B1

**Beschreibung**

Die Erfindung betrifft 5,6-Dihydro-11-alkylen-morphantridin-6-one, Verfahren zu ihrer Herstellung, diese enthaltende therapeutische Mittel und ihre Verwendung als Arzneimittel.

Es ist bekannt, daß tricyclische Ringsysteme vom Typ des 5,11-Dihydro-6H-pyrido [2.3-b] [1.4] benzodiazepin-6-ons wertvolle Eigenschaften besitzen (Arzneim.-Forsch. *27*, 356 (1977) ; DE-OS 27 25 501), die der Behandlung von Magen- und Duodenalulcera dienen. Weiter sind aus der DE-OS 27 24 478 in 11-Stellung substituierte 5,11-Dihydro-6H-pyrido [2,3-b] [1,4] benzodiazepin-6-one bekannt, die eine ulkushemmende und spasmolytische Wirkung besitzen. Schließlich beschreibt die EP-OS 19157 5,6-Dihydro-11-alkylen-morphantridin-6-one, die Zwischenprodukte zur Herstellung von Arzneimittelwirkstoffen darstellen.

Es wurde nun gefunden, daß 5,6-Dihydro-11-alkylen-morphantridin-6-one der Formel I

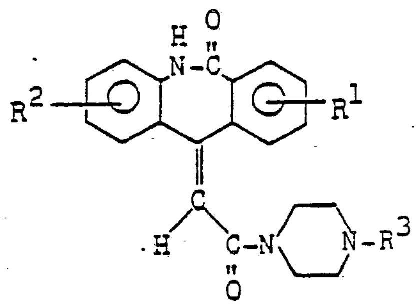

(I)

in der $R^1$ und $R^2$ Wasserstoff, Chlor oder Methyl und $R^3$ Wasserstoff oder $C_{1-3}$-Alkyl bedeuten und/oder deren physiologisch verträglichen Säureadditionssalze wertvolle pharmakologische Eigenschaften besitzen.

Die erfindungsgemäßen Verbindungen der Formel I können als cis-trans-Isomere Ia und Ib vorliegen :

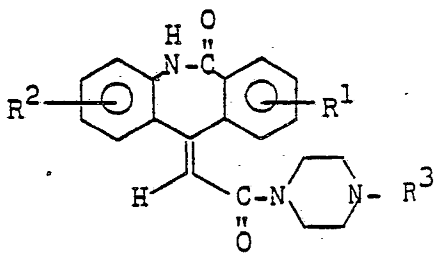

(Ia)

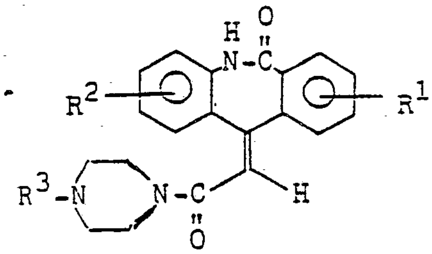

(Ib)

$R^3$ ist vorzugsweise ein Ethyl- und insbesondere ein Methylrest.

Die folgenden Verbindungen sind als besonders wirksam zu nennen :

cis,trans-11-(4-Methyl-piperazin-1-yl)-carbonylmethylen-5,6-dihydro-morphantridin-6-on
cis-11-(4-Methyl-piperazin-1-yl)-carbonylmethylen-5,6-dihydro-morphantridin-6-on
cis,trans-11-(4-Ethyl-piperazin-1-yl)-carbonylmethylen-5,6-dihydro-morphantridin-6-on
cis,trans-2-Chlor-11-(4-Ethyl-piperazin-1-yl)-carbonyl methylen-5,6-dihydro-morphantridin-6-on
cis,trans-2-Methyl-11-(4-methyl-piperazin-1-yl)-carbonyl methylen-5,6-dihydro-morphantridin-6-on.

Von diesen Verbindungen sind die cis-Isomeren bevorzugt.

Die erfindungsgemäßen Verbindungen der Formel I werden hergestellt, in dem man

a) eine Verbindung der Formel II

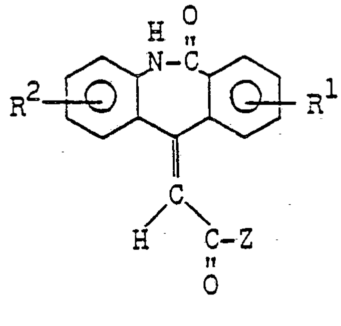

(II)

in der $R^1$ und $R^2$ die angegebenen Bedeutungen haben und Z eine nukleofuge Abgangsgruppe darstellt, mit einem Piperazin der Formel IIa

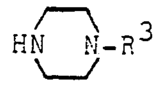

(IIa)

worin $R^3$ die angegebene Bedeutung hat, umsetzt oder

b) ein 5,6-Dihydro-morphantridin-6,11-dion der Formel V

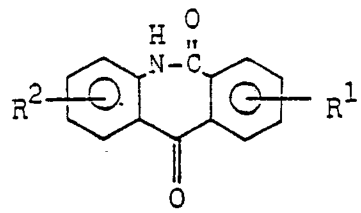

(V)

in der $R^1$ und $R^2$ die angegebenen Bedeutungen haben, mit einem Phosphonat der Formel VI

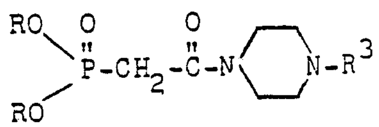

(VI)

in der R einen Alkylrest mit 1 bis 3 C-Atomen und $R^3$ die angegebene Bedeutung hat, unter den Bedingungen der Wittig-Reaktion in einem inerten Lösungsmittel in Gegenwart von einem Moläquivalent einer Base bei Temperaturen von 20 bis 80 °C umsetzt und die so erhaltene Verbindung gegebenenfalls in das reine cis- und trans-Isomere trennt und/oder gegebenenfalls in das Säureadditionssalz einer physiologisch verträglichen Säure überführt.

Als nukleofuge Abgangsgruppe für Z kommen Halogenatome, insbesondere Chlor, in Betracht.

Die Umsetzung a) erfolgt zweckmäßig in Gegenwart eines Moläquivalents eines tertiären Amins wie z. B. Triethylamin in einem inerten Lösungsmittel, wie einem cyclischen gesättigten Ether, insbesondere Tetrahydrofuran oder Dioxan, oder einem polaren aprotischen Lösungsmittel, vorzugsweise Dimethylformamid, bei Temperaturen von 0 bis 150 °C, bevorzugt 20 bis 80 °C, und ist im allgemeinen innerhalb von 3 bis 10 Stunden beendet.

Gegebenenfalls kann auch in Gegenwart einer überschüssigen Menge des verwendeten Amins der Formel IIa, das gleichzeitig als Lösungsmittel und gegebenenfalls als säurebindendes Mittel dient, gearbeitet werden.

Die Reaktion b) wird vorzugsweise in Dimethylformamid als Lösungsmittel durchgeführt. Als Base eignet sich besonders Natriumalkoholat, Natriumhydrid, Natriumamid oder eine metallorganische Verbindung, wie n-Butyl-lithium.

Die Verbindungen der Formel I werden in der Regel in Form von Kristallen erhalten und können durch Umkristallisation aus den üblichen organischen Lösungsmitteln, bevorzugt aus einem niederen Alkohol, wie Ethanol, oder einem niedrigen Ester, bevorzugt Essigsäurethylester, umkristallisiert oder durch Säulenchromatographie gereinigt werden.

Die Phosphonate der Formel VI können — soweit sie nicht bekannt sind — durch Arbusow-Reaktion aus dem entsprechenden Halogenalkylsäureamid und Trialkylphosphit hergestellt werden.

Das cis-trans-Isomerengemisch kann durch fraktionierte Kristallisation oder durch Säulenchromatographie getrennt werden. Die fraktionierte Kristallisation kann in einem niedrigen Ester, bevorzugt Essigsäureethylester, oder einem niederen einwertigen Alkohol, wie Methanol oder Ethanol, erfolgen. Die Trennung durch Säulenchromatographie gelingt insbesondere an Kieselgel, mit Methylenchlorid oder einer Mischung aus Methylenchlorid und Methanol im Volumenverhältnis 99 : 1 bis 85 : 15.

Die Zuordnung der einzelnen Isomeren erfolgt beispielsweise durch Röntgenstrukturanalyse.

Gegebenenfalls werden die erhaltenen erfindungsgemäßen Verbindungen in das Säureadditionssalz einer physiologisch verträglichen Säure überführt. Als übliche physiologisch verträglich organische oder anorganische Säuren kommen beispielsweise in Betracht Salzsäure. Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können beispielsweise dem Journal of Pharmaceutical Sciences, Volume 66, Seiten 1 bis 5 (1977) entnommen werden.

Die Säureadditionssalze werden in der Regel in an sich bekannter Weise z. B. durch Mischen der

freien Base oder deren Lösungen mit der entsprechenden Säure oder deren Lösungen in einem organischen Lösungsmittel, beispielsweise einem niederen Alkohol wie Methanol, Ethanol oder Propanol oder Methylisobutylketon oder einem Ether wie Diethylether, Tetrahydrofuran oder Dioxan, erhalten. Zur besseren Kristallabscheidung können auch Mischungen der genannten Lösungsmittel verwendet werden.

Die Ausgangsverbindungen der Formel II werden erhalten, indem man ein cis, trans-11-Carboxymethylen-5,6-dihydromorphantridin-6-on der Formel III

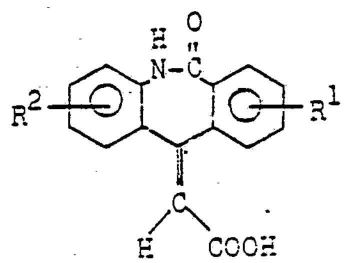

(III)

in der $R^1$ und $R^2$ die angegebenen Bedeutungen haben, in üblicher Weise mit überschüssigem Thionylchlorid bei Raumtemperatur in das Carbonsäurehalogenid überführt.

Die Verbindungen der Formel III werden durch Verseifung der entsprechenden Ester (DE-OS 29 18 832) mit alkoholischer Alkalilauge bei 40 bis 90 °C erhalten.

Die erfindungsgemäßen Verbindungen und ihre physiologisch verträglichen Säureadditionssalze sind Pharmaka, die als wertvolle Heilmittel bei der Behandlung von Krankheitszuständen geeignet sind, die mit einer pathologisch gesteigerten Magensekretion einhergehen — wie z. B. Magen- und Duodenalulcera.

Die Hemmung der Magensäuresekretion kommt in einem Anstieg des pH-Wertes der Magenschleimhaut zum Ausdruck. Sie wird an Gruppen von 5 wachen weiblichen Sprague-Dawley-Ratten (160-180 g) untersucht. Die Tiere erhalten 48 Stunden kein Futter (Wasser ad libitum) und werden mit unterschiedlichen Dosen der Prüfsubstanzen (s.c.) vorbehandelt. Nach 1 Stunde Werden sie mit Hexobarbital-Na (46,4 mg/kg i.v.) narkotisiert. Anschließend wird eine pH-Elektrode (Philips-Spezialelektrode Typ CJP) in den Magen eingeführt und der pH-Wert auf der Oberfläche der Magenschleimhaut gemessen (pH-Wert bei unbehandelten Tieren : 1,40 ± 0,02 ; N = 200). Aus der linearen Regression zwischen den Logarithmen der applizierten Dosen und der Zunahme des pH-Wertes wird als ED 0,75 die Dosis bestimmt, die im Vergleich zu unbehandelten Kontrolltieren eine pH-Zunahme um durchschnittlich 0,75 bewirkt.

Zur Untersuchung der antiulcerogenen Wirkung erhalten Gruppen von 10 weiblichen Sprague-Dawley-Ratten (160-180 g) 1 mg/kg Reserpin i.p. und bleiben danach 18 Stunden ohne Futter (Wasser ad libitum). Nach dieser Zeit erhalten die Tiere 21,5 mg/Kg Indomethacin i.p. und die Prüfsubstanz per os und werden anschließend 6 Stunden lang bei einer Temperatur von 8 °C gehalten und danach getötet. Die Mägen werden entnommen und die Fläche der ulcerösen. Schleimhautläsionen bestimmt. Aus den linearen Regressionen zwischen den Logarithmen der applizierten Dosen und der relativen Verkleinerung der Fläche der Ulcerationen bezogen auf die Kontrolltiere wird als ED 50 % die Dosis bestimmt, die eine Verkleinerung der Ulcusfläche um 50 % bewirkt.

Zur Feststellung anticholinergischer Nebenwirkungen (Mydriasis, Salivationshemmung) erhalten Gruppen von 5 bis 10 weiblichen Sprague-Dawley-Ratten (160-200 g) die Prüfsubstanz s.c. Nach 1 Stunde wird der Pupillendurchmesser gemessen. Anschließend erhalten die Tiere 0,6 mg/kg Carbachol i.p. Nach 5 Minuten wird der durch die Carbacholstimulation sezernierte Speichel mit Hilfe von 4 cm breiten Streifen Universal-Indikatorpapier (Merck) aufgesaugt. Als Maß für die Speichelsekretion dient die durch den alkalischen Speichel blaugefärbte Fläche des Indikatorpapiers. Eine Hemmung der Speichelsekretion durch die Prüfsubstanzen wird angenommen, wenn die blau gefärbte Fläche des Papiers um mehr als 50 % kleiner ist als bei den Kontrollen. Die Auswertung der linearen Beziehungen zwischen den Logarithmen der Dosen und der Häufigkeit der Salivationshemmung erfolgt alternativ durch Probitanalyse. Als ED 50 wird die Dosis bestimmt, die mit einer relativen Häufigkeit von 50 % eine Salivationshemmung verursacht.

Bei der mydriatischen Wirkung bestehen lineare Regressionen zwischen den Dosenlogarithmen und der Zunahme des Pupillendurchmessers in mm, aus dem als ED 2 mm die Dosis berechnet wird, die den Pupillendurchmesser um 2 mm vergrößert.

Als Referenzsubstanz dient Pirenzepin (5,11-Dihydro-11-[(4-methyl-1-piperazinyl)-acetyl]-6H-pyrido-[2,3,b] [-1,4]-benzodiazepin-6-on ; DE-PS 1 795 183).

Die erfindungsgemäßen Verbindungen bewirken eine Hemmung der Säuresekretion des Magens, die durch eine dosisabhängige Erhöhung des pH-Wertes auf der Oberfläche der Magenschleimhaut zum Ausdruck kommt (Tab. 1). Ferner hemmen sie die Bildung von Magenulcera (Tab. 2). In beiden Tests übertreffen die Substanzen das bekannte Pharmakon Pirenzepin an Wirksamkeit (DE-PS 1 795 183). Darüberhinaus besitzen sie eine höhere Wirkungs-Spezifität als Pirenzepin, die durch einen wesentlich

größeren Abstand zwischen den sekretionshemmenden Dosen und den Dosen, welche unerwünschte anticholinergische Nebenwirkungen wie Mydriasis oder Salivationshemmung hervorrufen, nachgewiesen wird (Tab. 1).

Tabelle 1

Hemmung der Magensekretion und anticholinergische Wirkung an der Ratte

| Testmodell | | Bsp. 1 cis/trans | Bsp. 1 cis | Pirenzepin |
|---|---|---|---|---|
| Hemmung der Magensäuresekretion | ED 0,75 | 0,055 | 0,032 | 0,086 |
| | R.W. | >1,6 | 2,7 | 1,0 |
| Mydriatische Wirkung | ED 2 mm | >215 | 198 | 1,6 |
| | ED 2 mm/ ED 0,75 | >3900 | 6200 | 19 |
| Salivationshemmende Wirkung | ED 50 | 99 | 50 | 2,0 |
| | ED 50/ ED 0,75 | 1800 | 1600 | 23 |

Appl. s.c. ; R.W. = Relative Wirksamkeit
Wirksame Dosen in mg/kg

Tabelle 2

Antiulcerogene Wirkung an der Ratte

| Beispiel | ED 50 % mg/kg | R.W. |
|---|---|---|
| 1 cis/trans | 2,1 | 3,1 |
| 1 cis | 1,7 | 3,8 |
| Pirenzepin | 6,5 | 1,0 |

App. : per os ; R.W. = Relative Wirksamkeit

Gegenstand der vorliegenden Erfindung sind daher weiter Arzneimittel, die eine Verbindung der Formel I oder deren physiologisch verträgliches Säureadditionssalz enthalten, sowie die Verwendung der neuen Verbindungen bei der Behandlung von Krankheitszuständen, die mit einer pathologisch gesteigerten Magensekretion einhergehen.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmittel, Dispergiermitteln, Emulgatoren und/oder Konservierungsmitteln verarbeitet werden (vgl. H. Sucker et al : Pharmazeutische Technologie, Thieme-Verlag, Stuttgart, 1978). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Infusionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit Polyvinylpyrrolidon, Calciumcarbonat, Calciumphosphat oder Milchzucker, Sprengmittel wie Mais, Stärke oder Alginsäure, Bindemittel wie Stärke oder Gelatine, Gleitmittel wie Magnesiumstearat oder Talk und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können, außerdem Suspendierstoffe, die Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyethylenglykol bzw. deren Derivaten herstellen.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 100, vorzugsweise 10 bis 80 mg.

Die vorliegende Erfindung wird durch die nachfolgenden Ausführungsbeispiele näher erläutert. Die Schmelzpunkte der cis, trans-Isomerengemische können dabei in Abhängigkeit vom cis-trans-Mengenverhältnis Schwankungen unterliegen.

## Beispiel 1

cis- und trans-11-(4-Methylpiperazin-1-yl)-carbonyl-methylen-5,6-dihydromorphantridin-6-on

### A. Herstellung des Ausgangsproduktes

30,0 g (108 mM) 11-Carbomethoxymethylen-5,6-dihydromorphantridin-6-on in 150 ml Ethanol werden mit 200 ml 10 %iger Natronlauge versetzt und 2 Stunden bei Rückflußtemperatur gerührt. Nach dem Abkühlen filtriert man das Reaktionsgemisch und engt im Wasserstrahlvakuum etwa auf die Hälfte ein. Anschließend stellt man unter Eiskühlung mit konz. Salzsäure sauer und saugt die ausfallenden Kristalle unter gutem Nachwaschen mit Wasser ab. Man isoliert 27 g (94 %) cis, trans-11-Carboxymethylen-5,6-dihydromorphantridin-6-on, Schmp. 258-260 °C.

31,0 g (124 mM) der so erstellten Verbindung werden mit 200 ml Thionylchlorid versetzt und bei Raumtemperatur gerührt. Innerhalb 1 Stunde tritt Lösung ein. Nach weiterem einstündigem Rühren zeiht man das Thionylchlorid im Ölpumpenvakuum ab, nimmt den Rückstand mit wenig Toluol auf und zieht das Lösungsmittel wiederum vollständig ab. Das verbleibende cis, trans-11-Chlorcarbonylmethylen-5,6-dihydromorphantridin-6-on (Ausbeute 99 %) ist genügend rein für die weitere Umsetzung.

### B. Herstellung des Endproduktes

35,1 g (124 mM) 11-Chlorcarbonylmethylen-5,6-dihydromorphantridin-6-on (cis, trans-Isomerengemisch) in 140 ml Dimethylformamid wird unter gutem Rühren portionsweise mit 12,9 g (129 mM) N-Methylpiperazin und 12,5 g (125 mM) Treithylamin versetzt und 2 Stunden unter Stickstoffatmosphäre bei Raumtemperatur gerührt. Nach vollständigem Abdestillieren des Lösungsmittels im Vakuum verteilt man den Rückstand zwischen Methylenchlorid und Wasser, macht die wäßrige Phase mit verdünnter Natronlauge schwach alkalisch, extrahiert die wäßrige Phase noch zweimal mit Methylenchlorid und wäscht die vereinigten organischen Phasen mit Wasser gründlich nach. Trocknen und Einengen der organischen Phase liefert 36 g Rohprodukt.

Zur Herstellung des reinen cis, trans-Isomerengemisches reinigt man das Rohprodukt durch Säulenchromatographie (Kieselgel, Methylenchlorid/Methanol 95/5). Man isoliert 34,0 g (75 %) cis, trans-11-(4-Methyl-piperazin-1-yl)-carbonylmethylen-5,6-dihydromorphantridin-6-on. $H_2O$, Schmp. 125-128 °C.

Zur Trennung der cis, trans-Isomeren digeriert man 16,0 g des Isomerengemisches in 200 ml siedendem Essigsäureethylester und saugt die unlöslichen Anteile in der Hitze ab. Man erhält 7,0 g farblose Festkörper, die nach dem Dünnschichtchromatogram (Kieselgel, Laufmittel Toluol/Methanol 85/15) vornehmlich aus dem unpolaren cis-Isomeren bestehen, und aus Essigsäureethylester umkristallisiert werden, Schmp. 229-231°C.

Nach dem Einengen des Filtrats nimmt man den Rückstand in soviel siedendem Essigsäureethylester auf, bis gerade alles in Lösung geht. Die in der Kälte kristallisierende 1. Fraktion besteht häufig aus dem

Isomerengemisch. Danach kristallisiert jedoch aus der verbleibenden Mutterlauge ca. 5 g des stark angereicherten polaren trans-Isomeren.

Durch nochmaliges Umkristallisieren aus Essigsäureethylester erhält man das reine trans-Isomere in Form farbloser Kristalle, Schmp. 219-221 °C.

Beide Isomeren enthalten 1/4 Mol Kristallwasser. Die Zuordnung der beiden geometrischen Isomeren erfolgte mit Hilfe der Röntgenstrukturanalyse.

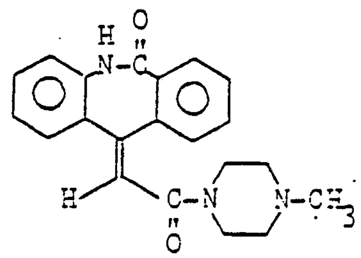

cis

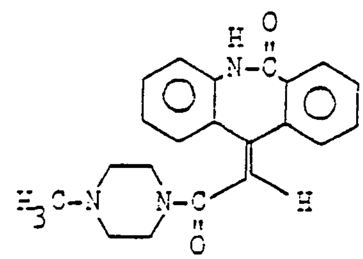

trans

Analog Beispiel 1 wurden hergestellt :

2. cis, trans-11-(4-Ethylpiperazin-1-yl)-carbonylmethylen-5,6-dihydromorphantridin-6-on · 0,5 $H_2O$, Schmp. 112-115 °C.

3. cis, trans-2-Methyl-11-(4-methylpiperazin-1-yl)-carbonylmethylen-5,6-dihydromorphantridin-6-on · 0,5$H_2O$, Schmp. 115-117 °C.

4. cis, trans-2-Methyl-11-(4-ethylpiperazin-1-yl)-carbonylmethylen-5,6-dihydromorphantridin-6-on · 0,5$H_2O$, Schmp. 116-117 °C.

5. cis, trans-3-Methyl-11-(4-methylpiperazin-1-yl)-carbonylmethylen-5,6-dihydromorphantridin-6-on · 0,5 $H_2O$. 0,5 HCl, Schmp. 117-118 °C.

6. cis, trans-3-Methyl-11-(4-ethylpiperazin-1-yl)-carbonylmethylen-5,6-dohydromorphantridin-6-on · $H_2O$, Schmp. 216-220 °C.

7. cis, trans-2-Chlor-11-(4-methylpiperazin-1-yl)-carbonylmethylen-5,6-dihydromorphantridin-6-on · 0,5$H_2O$, Schmp. 230-232 °C.

8. cis, trans-2-Chlor-11-(4-ethylpiperazin-1-yl)-carbonylmethylen-5,6-dihydromorphantridin-6-on, Schmp. 230-233 °C.

### Beispiel 9

cis, trans-11-Piperazin-1-yl-carbonylmethylen-5,6-dihydromorphantridin-6-on · 2 $H_2O$

7,0 g (25 mM) 11-Chlorcarbonylmethylen-5,6-dihydromorphantridin-6-on (cis, trans-Isomerengemisch) in 50 ml Dimethylformamid wird unter gutem Rühren zu einer Lösung aus 7,8 g (91 mM) Piperazin in 50 ml Dimethylformamid zugetropft und 3 Stunden unter Stickstoffatmosphäre bei Raumtemperatur gerührt. Nach vollständigem Abdestillieren des Lösungsmittels am Ölpumpenvakuum verteilt man den Rückstand zwischen Methylenchlorid und Wasser, stellt die wäßrige Phase mit verdünnter Natronlauge schwach alkalisch, extrahiert die wäßrige Phase noch zweimal mit Methylenchlorid und wäscht die vereinigten organischen Phasen mit Wasser gründlich nach. Trocknen und Einengen der organischen Phase liefert das Rohprodukt.

Zur Herstellung des reinen cis, trans-Isomerengemisches reinigt man das Rohprodukt durch Säulenchromatographie (Kieselgel, Laufmittel Methylenchlorid/Methanol 95/5). So erhält man 31 % farblose Kristalle, Schmp. 150-152 °C (Zers.).

### Beispiel 10

cis, trans-11-(4-Methylpiperazin-1-yl-carbonylmethylen-5,6-dihydromorphantridin-6-on · $H_2O$

A. Herstellung des Wittig-Phosphonates

20,0 g (177 mM) Chloracetylchlorid in 200 ml Methylenchlorid wird bei 0 °C unter gutem Rühren mit 17,7 g (177 mM) N-Methylpiperazin versetzt und 2 Stunden bei Raumtemperatur weitergerührt. Man macht das Reaktionsgemisch anschließend mit 10 % Natronlauge schwach alkalisch, läßt noch 15 Min. bei Raumtemperatur nachrühren, trennt die organische Phase ab und extrahiert die wäßrige Phase noch zweimal mit Methylenchlorid. Die vereinigten organischen Phasen werden mit Wasser gewaschen, getrocknet und auf ca. 200 ml eingeengt.

Zu dieser Lösung von 1-Chloracetyl-4-methyl-piperazin in Methylenchlorid gibt man 147 g (885 mM)

Triethylphosphit und destilliert über eine Füllkörperkolonne das Methylenchlorid bei Normaldruck ab. Anschließend erhitzt man unter Beibehaltung der Kolonnendestillation 2 Stunden lang auf 140 °C (Abdestillieren von Ethylenchlorid). Nach vorsichtigem Abdestillieren des Triethylphosphits im Ölpumpenvakuum wird der Rückstand durch Säulenchromatographie (Kieselgel, Laufmittel Methylenchlorid/Methanol 95/5) gereinigt. Man isoliert 15 g (31 %) Diethyl-4-methylpiperazin-1-yl-phosphonoacetamid als gelbes Öl.

B. Herstellung des Endproduktes

7,2 g (32,4 mM) 5,6-Dihydromorphantridin-6,11-dion werden in 40 ml Dimethylformamid gelöst und unter Stickstoff gerührt. Man läßt dann gleichzeitig 13,3 g (48,0 mM) Diethyl-4-methylpiperazin-1-yl-phosphonoacetamid und 8,4 g (48,0 mM) Natriummethylat (30 %) gelöst in 20 ml Dimethylformamid langsam zutropfen (Farbzunahme und Temperaturerhöhung zeigen den Beginn der Wittig-Reaktion an). Nach 12-stündigem Nachrühren bei Raumtemperatur entfernt man das Lösungsmittel im Ölpumpenvakuum und extrahiert den Rückstand zwischen Wasser und Methylenchlorid. Nach Einengen der organischen Phase reinigt man das Rohprodukt durch Säulenchromatographie (Kieselgel, Methylenchlorid/Methanol 95/5). Man isoliert 4,1 g (35 %) cis, trans-11-(4-Methyl-piperazin-1-yl)-carbonylmethylen-5,6-dihydromorphantridin-6-on · $H_2O$, Schmp. 111-115 °C.

**Patentansprüche** (für die Vertragsstaaten : BE, CH, DE, FR, GB, IT, LI, LU, NL, SE)

1. 5,6-Dihydro-11-alkylen-morphantridin-6-one der Formel I

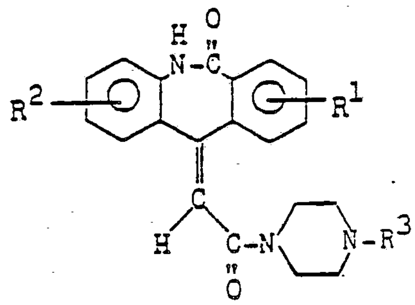

(I)

in der $R^1$ und $R^2$ Wasserstoff, Chlor oder Methyl und $R^3$ Wasserstoff oder $C_{1-3}$-Alkyl bedeuten und/oder deren physiologisch verträglichen Säureadditionssalze.

2. Verbindungen der Formel I gemäß Anspruch 1 in der cis-Form.

3. 11-(4-Methylpiperazin-1-yl)-carbonylmethylen-5,6-dihydromorphantridin-6-on.

4. cis-11-(4-Methylpiperazin-1-yl)-carbonylmethylen-5,6-dihydromorphantridin-6-on.

5. Arzneimittel enthaltend eine Verbindung der Formel I gemäß Anspruch 1.

6. Verbindungen der Formel I gemäß Anspruch 1 zur Verwendung bei der Bekämpfung von Ulcera.

7. Verfahren zur Herstellung der Verbindungen der Formel I gemäß Anspruch 1, dadurch gekennzeichnet, daß man

a) eine Verbindung der Formel II

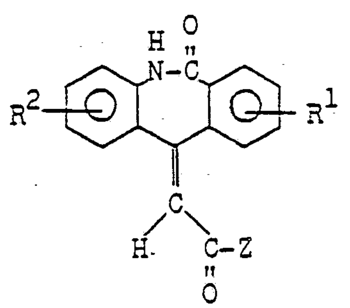

(II)

in der $R^1$ und $R^2$ die angegebenen Bedeutungen haben und Z eine nukleofuge Abgangsgruppe darstellt, mit einem Piperazin der Formel IIa

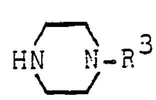

(IIa)

worin $R^3$ die angegebene Bedeutung hat, umsetzt oder

b) ein 5,6-Dihydromorphantridin-6,11-dion der Formel V

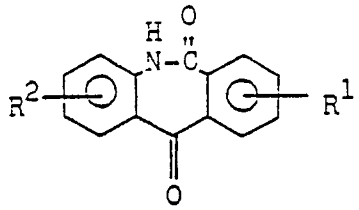

(V)

in der $R^1$ und $R^2$ die angegebenen Bedeutungen haben, mit einem Phosphonat der Formel VI

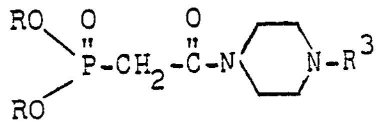

(VI)

in der R einen Alkylrest mit 1 bis 3 C-Atomen und $R^3$ die angegebene Bedeutung hat, unter den Bedingungen der Wittig-Reaktion in einem inerten Lösungsmittel in Gegenwart von einem Moläquivalent einer Base bei Temperaturen von 20 bis 80 °C umsetzt und die so erhaltene Verbindung gegebenenfalls in das reine cis- und trans-Isomere trennt und/oder gegebenenfalls in das Säureadditionssalz einer physiologisch verträglichen Säure überführt.

**Patentansprüche** (für den Vertragsstaat AT)

1. Verfahren zur Herstellung von 5,6-Dihydro-11-alkylen-morphantridin-6-onen der Formel I

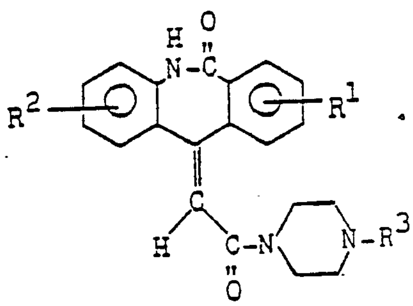

(I)

in der $R^1$ und $R^2$ Wasserstoff, Chlor oder Methyl und $R^3$ Wasserstoff oder $C_{1-3}$-Alkyl bedeuten, und/oder deren physiologisch verträglichen Säureadditionssalzen, dadurch gekennzeichnet, daß man

a) eine Verbindung der Formel II

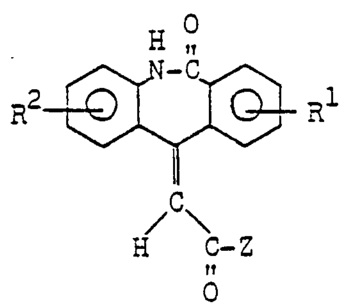

(II)

in der $R^1$ und $R^2$ die angegebenen Bedeutungen haben und Z eine nukleofuge Abgangsgruppe darstellt, mit einem Piperazin der Formel IIa

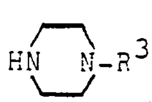

(IIa)

worin $R^3$ die angegebene Bedeutung hat, umsetzt oder

b) ein 5,6-Dihydro-morphantridin-6,11-dion der Formel V

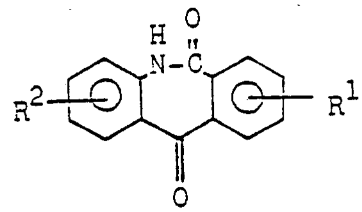

(V)

in der $R^1$ und $R^2$ die angegebenen Bedeutungen haben, mit einem Phosphonat der Formel VI

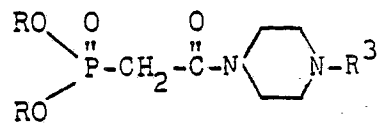

(VI)

in der R einen Alkylrest mit 1 bis 3 C-Atomen und $R^3$ die angegebene Bedeutung hat, unter den Bedingungen der Wittig-Reaktion in einem inerten Lösungsmittel in Gegenwart von einem Moläquivalent einer Base bei Temperaturen von 20 bis 80 °C umsetzt und die so erhaltene Verbindung gegebenenfalls in das reine cis- und trans-Isomere trennt und/oder gegebenenfalls in das Säureadditionssalz einer physiologisch verträglichen Säure überführt.

2. Verfahren gemäß Anspruch 1, dadurch gekennzeichnet, daß man die Verbindungen der Formel I in der cis-Form herstellt.

3. Verfahren gemäß Anspruch 1, dadurch gekennzeichnet, daß man 11-(4-Methylpiperazin-1-yl)-carbonyl-methylen-5,6-dihydromorphantridin-6-on herstellt.

4. Verfahren gemäß Anspruch 1, dadurch gekennzeichnet, daß man cis-11-(4-Methylpiperazin-1-yl)-carbonyl-methylen-5,6-dihydromorphantridin-6-on herstellt.

**Claims** (for the Contracting States : BE, CH, DE, FR, GB, IT, LI, LU, NL, SE)

1. A 5,6-dihydro-11-alkylene-morphanthridin-6-one of the formula I

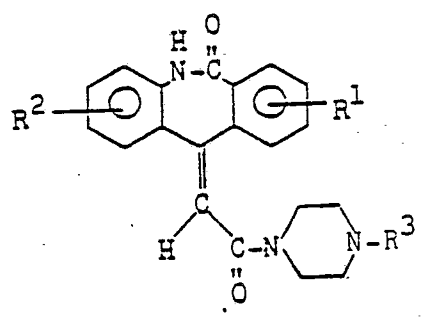

(I)

where $R^1$ and $R^2$ are each hydrogen, chlorine or methyl, and $R^3$ is hydrogen or alkyl of 1 to 3 carbon atoms, and/or its physiologically tolerated acid addition salts.

2. A compound of the formula I as claimed in claim 1, in the cis-form.

3. 11-(4-Methylpiperazinyl-1-yl)-carboxamidomethylene-5,6-dihydromorphanthridin-6-one.

4. cis-11-(4-Methylpiperazinyl-1-yl)-carboxamidomethylene-5,6-dihydromorphanthridin-6-one.

5. A drug which contains a compound of the formula I as claimed in claim 1.

6. A compound of the formula I as claimed in claim 1 for use in the control of ulcers.

7. A process for the preparation of a compound of the formula I as claimed in claim 1, wherein

a) a compound of the formula II

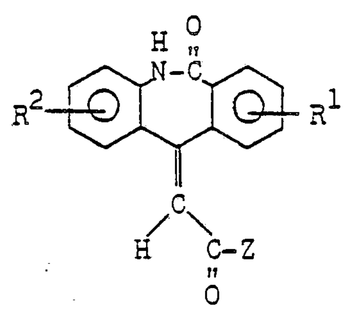

(II)

where $R^1$ and $R^2$ have the stated meanings and Z is a nucleofugic leaving group, is reacted with a piperazine of the formula IIa

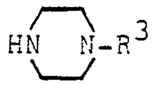

(IIa)

where $R^3$ has the stated meanings, or

b) a 5,6-dihydro-morphanthridine-6,11-dione of the formula V

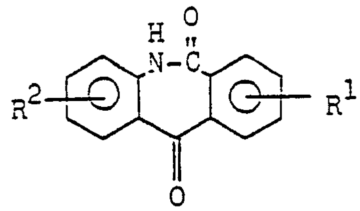

(V)

where $R^1$ and $R^2$ have the stated meanings, is reacted with a phosphonate of the formula VI

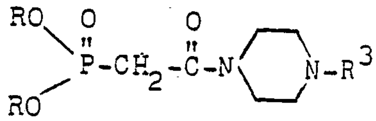

(VI)

where R is alkyl of 1 to 3 carbon atoms, and $R^3$ has the stated meanings, under the conditions of a Wittig reaction, in an inert solvent in the presence of one mole equivalent of a base, at from 20 to 80 °C, and, if desired, the resulting compound is separated into the pure cis-isomer and trans-isomer and/or is converted into an addition salt with a physiologically tolerated acid.

**Claims** (for the Contracting State AT)

1. A process for the preparation of a 5,6-dihydro-11-alkylene-morphanthridin-6-one of the formula I

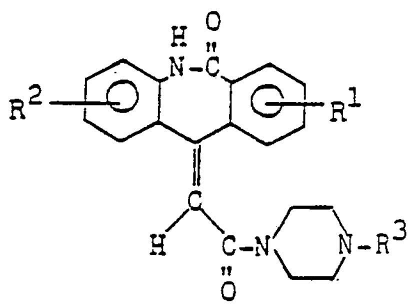

(I)

where $R^1$ and $R^2$ are each hydrogen, chlorine or methyl, and $R^3$ is hydrogen or alkyl of 1 to 3 carbon atoms, and/or its physiologically tolerated acid addition salts, wherein

a) a compound of the formula II

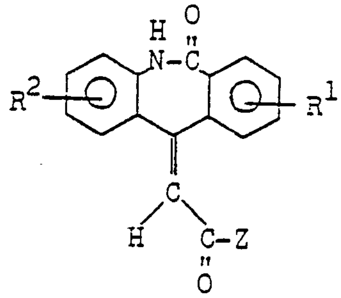

(II)

where $R^1$ and $R^2$ have the stated meanings and Z is a nucleofugic leaving group, is reacted with a piperazine of the formula IIa

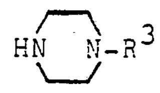

(IIa)

where $R^3$ has the stated meanings, or

b) a 5,6-dihydro-morphanthridine-6,11-dione of the formula V

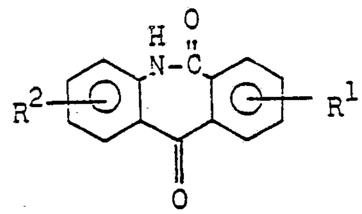

(V)

where $R^1$ and $R^2$ have the stated meanings, is reacted with a phosphonate of the formula VI

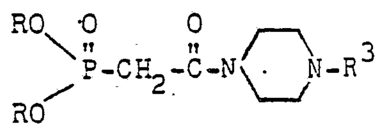

(VI)

where R is alkyl of 1 to 3 carbon atoms, and $R^3$ has the stated meanings, under the conditions of a Wittig reaction, in an inert solvent in the presence of one mole equivalent of a base, at form 20 to 80 °C, and, if desired, the resulting compound is separated into the pure cis-isomer and trans-isomer and/or is converted into an addition salt with a physiologically tolerated acid.

2. A process as claimed in claim 1, wherein a compound of the formula I is prepared in the cis-form.

3. A process as claimed in claim 1, wherein 11-(4-methyl-piperazinyl-1-yl)-carboxamidomethylene-5,6-dihydromorphanthridin-6-one is prepared.

4. A process as claimed in claim 1, wherein cis-11-(4-methylpiperazinyl-1-yl)-carboxamidomethylene-5,6-dihydromorphanthridin-6 one is prepared.

**Revendications** (pour les Etats contractants : BE, CH, DE, FR, GB, IT, LI, LU, NL, SE)

1. 5,6-dihydro-11-alkylèn-morphantridin-6-ones de formule I

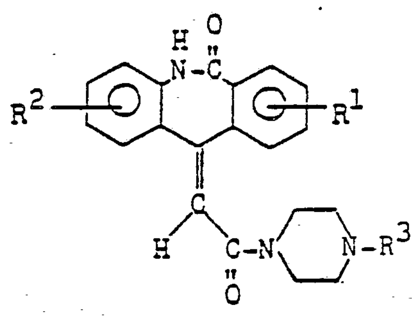

(I)

dans laquelle $R^1$ et $R^2$ représentent hydrogène, chlore ou méthyle et $R^3$ hydrogène ou alkyle en $C_{1-3}$ et/ou leurs sels d'addition d'acide acceptables physiologiquement.

2. Composés de formule I selon la revendication 1, dans la forme cis.

3. 11-(4-méthylpipérazin-1-yl)-carbonylméthylèn-5,6-dihydromorphantridin-6-one.

4. cis-11-(4-méthylpipérazin-1-yl)-carbonylméthylèn-5,6-dihydromorphantridin-6-one.

5. Médicament contenant un composé de formule I selon la revendication 1.

6. Composés de formule I selon la revendication 1, pour utilisation dans la lutte contre les ulcères.

7. Procédé de préparation des composés de formule I selon la revendication 1, caractérisé par le fait que l'on fait réagir, dans les conditions de la réaction de Wittig, dans un solvant inerte, en présence d'un équivalent molaire d'une base, à des températures de 20 à 80 °C,

a) un composé de formule II

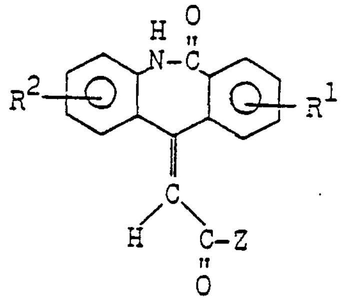

(II)

dans laquelle $R^1$ et $R^2$ ont les significations indiquées et Z représente un groupe éliminable nucléofuge, avec une pipérazine de formule IIa

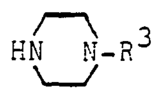

(IIa)

où $R^3$ a la signification indiquée, ou

b) une 5,6-dihydro-morphantridin-6,11-dione de formule V

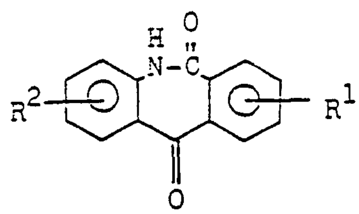

(V)

dans laquelle $R^1$ et $R^2$ ont les significations indiquées, avec un phosphonate de formule VI

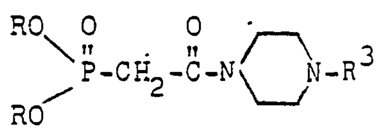

(VI)

dans laquelle R est un reste alkyle de 1 à 3 atomes C et $R^3$ a la signification indiquée, et l'on sépare éventuellement le composé ainsi obtenu, en les isomères cis- et trans- purs et/ou le transforme éventuellement en le sel d'addition d'un acide physiologiquement acceptable.

**Revendications** (pour l'Etat contractant AT)

1. Procédé de préparation de 5,6-dihydro-11-alkylèn-morphantridin-6-ones de formule I

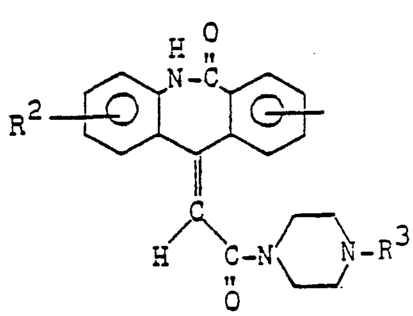

(I)

dans laquelle $R^1$ et $R^2$ représentent hydrogène, chlore ou méthyle et $R^3$ hydrogène ou alkyle en $C_{1-3}$ et/ou leurs sels d'addition d'acide acceptables physiologiquement, caractérisé par le fait qu'on fait réagir, dans les conditions de la réaction de Wittig, dans un solvant inerte, en présence d'un équivalent molaire d'une base, à des températures de 20 à 80 °C,

a) un composé de formule II

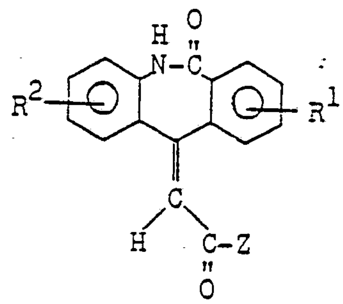

(II)

dans laquelle $R^1$ et $R^2$ ont les significations indiquées et Z représente un groupe éliminable nucléofuge, avec une pipérazine de formule IIa

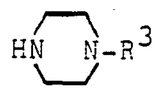

(IIa)

où $R^3$ a la signification indiquée, ou

b) une 5,6-dihydro-morphantridin-6,11-dione de formule V

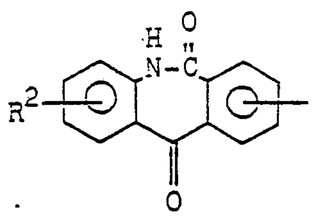

(V)

dans laquelle $R^1$ et $R^2$ ont les significations indiquées, avec un phosphonate de formule VI

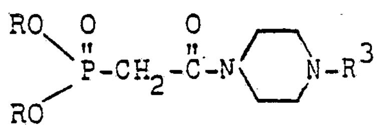

(VI)

dans laquelle R est un reste alkyle de 1 à 3 atomes C et $R^3$ a la signification indiquée, et l'on sépare éventuellement le composé ainsi obtenu, en les isomères cis- et trans- purs et/ou le transforme éventuellement en le sel d'addition d'un acide physiologiquement acceptable.

2. Procédé selon la revendication 1, caractérisé par le fait qu'on prépare le composé de formule I sous la forme cis-.

3. Procédé selon la revendication 1, caractérisé par le fait qu'on prépare la ll-(4-méthylpipérazin-1-yl)-carbonyl-méthylèn-5,6-dihydromorphantridin-6-one.

4. Procédé selon la revendication 1, caractérisé par le fait qu'on prépare la cis-11-(4-méthylpipérazin-1-yl)-carbonylméthylèn-5,6-dihydromorphantridin-6-one.